# EUROPÄISCHE PATENTSCHRIFT

(11) **EP 4 595 885 B1**
(45) Veröffentlichungstag und Bekanntmachung des Hinweises auf die Patenterteilung: **16.09.2026**
(21) Anmeldenummer: 24154923.7
(22) Anmeldetag: 31.01.2024
(51) Int. Cl.: A61B 6/00, A61B 6/12, A61B 6/50

(54) **RÖNTGENBILDGEBUNGSVERFAHREN, DATENVERARBEITUNGSVORRICHTUNG, RÖNTGENBILDGEBUNGSSYSTEM UND COMPUTERPROGRAMMPRODUKT**
RADIOGRAPHIC IMAGING METHOD, DATA PROCESSING APPARATUS, RADIOGRAPHIC IMAGING SYSTEM, AND COMPUTER PROGRAM PRODUCT
PROCÉDÉ D'IMAGERIE PAR RAYONS X, DISPOSITIF DE TRAITEMENT DE DONNÉES, SYSTÈME D'IMAGERIE PAR RAYONS X ET PRODUIT DE PROGRAMME INFORMATIQUE

(43) Veröffentlichungstag der Anmeldung: 06.08.2025
(73) Patentinhaber: Siemens Healthineers AG, 91301 Forchheim (DE)
(72) Erfinder: Manhart, Michael, 90765 Fürth (DE)
(74) Vertreter: Siemens Healthineers Patent Attorneys

(56) Entgegenhaltungen:
- US-A1- 2018 168 532
- US-A1- 2020 410 666
- US-A1- 2023 380 787

## Beschreibung

Die vorliegende Erfindung betrifft ein Röntgenbildgebungsverfahren, wobei wenigstens zwei Röntgenbilder eines abzubildenden Bereichs eines Objekts erhalten werden, wobei die wenigstens zwei Röntgenbilder unterschiedlichen ersten Aufnahmezeiträumen entsprechen und eine Gefäßstruktur des Objekts in unterschiedlichen Kontrastmittelfüllzuständen darstellen. Die Erfindung betrifft ferner eine Datenverarbeitungsvorrichtung zur Durchführung eines solchen Röntgenbildgebungsverfahrens, ein entsprechendes Röntgenbildgebungssystem sowie ein entsprechendes Computerprogrammprodukt.

Bei Gefäßinterventionen unter Röntgenkontrolle können Livebildern während der Gefäßintervention Gefäßmasken überlagert werden, um eine entsprechende Gefäßstruktur für das behandelnde Personal in dem resultierenden Überlagerungsbild hervorzuheben, beispielsweise um das Personal bei der Führung eines medizinischen Instruments in der Gefäßstruktur zu unterstützen. Hierzu können insbesondere Verfahren zur digitalen Subtraktionsangiographie, DSA, und/oder Roadmap Verfahren durchgeführt werden.

Ein Problem dabei sind Bewegungen des abzubildenden Objekts, beispielsweise Patienten, verursacht etwa durch die Atmung des Patienten und/oder Organbewegungen, welche die Genauigkeit der Überlagerung reduzieren.

Eine Möglichkeit, dem zu begegnen, ist es, den Patienten entsprechend anzuweisen, bei der Aufnahme von Röntgenbildern die Luft anzuhalten. Dies ist jedoch zum einen unangenehm für den Patienten und zum anderen vergleichsweise unzuverlässig. Außerdem können Bewegungen aufgrund des Herzschlags oder sonstiger Organbewegungen dadurch nicht verhindert werden.

Dokument DE 10 2021 208 272 A1 schlägt ein Verfahren zum Erzeugen eines Subtraktionsbilds für eine DSA zur Reduktion von Rauschen und von Bewegungsartefakten vor. Dabei werden mehrere Maskenbilder eines Objekts vor einer Kontrastmittelgabe in das Objekt sowie ein Abbild des Objekts nach der Kontrastmittelgabe in das Objekt gewonnen. Ein erstes Summenbild wird aus den mehreren Maskenbildern gewonnen, indem die mehreren Maskenbilder jeweils multipliziert mit einem individuellen Gewicht summiert werden. Die individuellen Gewichte für jedes der mehreren Maskenbilder werden durch ein Optimierungsverfahren automatisch bestimmt und das Subtraktionsbild wird durch Subtraktion des Summenbilds von dem Abbild ermittelt.

Dadurch wird allerdings nur die Bewegung während der Erzeugung der Maskenbilder adressiert, nicht jedoch Bewegungen während der Erzeugung von mehreren Abbildern oder Livebildern nacheinander.

In der Veröffentlichung von O. Ronneberger et al: "U-Net: Convolutional Networks for Biomedical Image Segmentation" (arXiv:1505.04597) wird die U-Net-Architektur beschrieben, eine weit verbreitete CNN-Architektur zur Segmentierung von Bildern, die jedoch auch für andere Computer-Vision-Aufgaben, wie Objekterkennung et cetera, verwendet werden kann.

In der Veröffentlichung J. Chen et al.: "TransUNet: Transformers Make Strong Encoders for Medical Image Segmentation" (arXiv:2102.04306) wird TransUNet beschrieben, das zur Segmentierung medizinischer Bilder Transformer-Netzwerke mit U-Net vereint.

In der Veröffentlichung H. Wang et al.: "Mixed Transformer U-Net For Medical Image Segmentation" wird ein Transformer-Modul namens Mixed-Transformer-Module, MTM, zur Segmentierung medizinischer Bilder beschrieben.

Die US 2020/410666 A1 beschreibt ein System und Verfahren zur Erstellung bewegungskompensierter Bilder eines Patienten bei einem interventionellen medizinischen Eingriff.

Die US 2023/0380787 A1 offenbart die Verwendung von Gefäßbildern, die mit einem medizinischen Bildgebungsverfahren gewonnen wurden, als dynamische Gefäßkartierungen während eines interventionellen medizinischen Eingriffs.

Es ist eine Aufgabe der vorliegenden Erfindung, die Auswirkungen von Bewegungen eines abzubildenden Objekts zu reduzieren, wenn mehreren, in unterschiedlichen Aufnahmezeiträumen aufgenommenen, Livebildern Gefäßmasken überlagert werden sollen.

Diese Aufgabe wird durch den Gegenstand des unabhängigen Anspruchs gelöst. Vorteilhafte Weiterbildungen und bevorzugte Ausführungsformen sind Gegenstand der abhängigen Ansprüche. Die Erfindung beruht auf der Idee, basierend auf wenigstens zwei Röntgenbildern eine gemeinsame Gefäßmaske zu erzeugen, und basierend auf der gemeinsamen Gefäßmaske für jedes von wenigstens zwei Livebildern ein entsprechendes Überlagerungsbild zur Anzeige auf einem Anzeigegerät zu erzeugen.

Gemäß einem Aspekt der Erfindung wird ein Röntgenbildgebungsverfahren wie in Anspruch 1 angegeben.

Das Röntgenbildgebungsverfahren kann rein computerimplementiert sein. Sofern nicht anders angegeben, können alle Schritte des computerimplementierten Verfahrens von einer Datenverarbeitungsvorrichtung durchgeführt werden, die mindestens eine Recheneinheit beinhaltet. Insbesondere ist die mindestens eine Recheneinheit dazu eingerichtet oder angepasst, die Schritte des computerimplementierten Verfahrens auszuführen. Zu diesem Zweck kann die mindestens eine Recheneinheit beispielsweise ein Computerprogramm speichern, das Befehle beinhaltet, die, wenn sie von der mindestens einen Recheneinheit ausgeführt werden, die mindestens eine Recheneinheit dazu veranlassen, das computerimplementierte Verfahren auszuführen.

Für den Fall, dass die mindestens eine Recheneinheit zwei oder mehr Recheneinheiten beinhaltet, können bestimmte von der mindestens einen Recheneinheit durchgeführte Schritte auch so verstanden werden, dass verschiedene Recheneinheiten verschiedene Schritte oder verschiedene Teile eines Schrittes durchführen. Insbesondere ist es nicht erforderlich, dass jede Recheneinheit die Schritte vollständig durchführt. Mit anderen Worten kann die Durchführung der Schritte auf die zwei oder mehr Recheneinheiten verteilt werden.

Aus jeder Ausführungsform eines solchen computerimplementierten Verfahrens ergibt sich eine entsprechende Ausführungsform eines Röntgenbildgebungsverfahrens, das nicht rein computerimplementiert ist, indem entsprechende Schritte zur Erzeugung der wenigstens zwei Röntgenbilder und/oder der wenigstens zwei Livebilder einbezogen werden.

Sofern nicht anders angegeben, kann ein Bild hier und im Folgenden als Röntgenprojektionsbild verstanden werden oder als vorverarbeitetes Röntgenprojektionsbild oder als Kombination von Röntgenprojektionsbildern oder vorverarbeiteten Röntgenprojektionsbildern.

Die ersten Aufnahmezeiträume sind insbesondere aufeinanderfolgende Aufnahmezeiträume, die beispielsweise dieselbe Dauer aufweisen können. Jedes der wenigstens zwei Röntgenbilder wird dabei während eines der ersten Aufnahmezeiträume erzeugt, insbesondere mittels eines Röntgenbildgebungssystems.

Bei den wenigstens zwei Röntgenbildern kann es sich beispielsweise um Kontrastmittelbilder handeln, also um Bilder, die nach oder während der Verabreichung eines Kontrastmittels erzeugt wurden. Bei den wenigstens zwei Röntgenbildern kann es sich auch um Subtraktionsbilder handeln, die insbesondere anhand einer DSA erzeugt wurden. Hierbei werden entsprechende Kontrastmittelbilder beispielsweise mit einem oder mehreren Maskenbildern, die ohne Kontrastmittelverabreichung erzeugt werden, verrechnet, um die Gefäßstruktur deutlicher hervorzuheben.

Aufgrund der zeitlichen Dynamik der Verteilung des Kontrastmittels in der Gefäßstruktur zeigt jedes der wenigstens zwei Röntgenbilder einen anderen Kontrastmittelfüllzustand der Gefäßstruktur. Insbesondere können in verschiedenen der wenigstens zwei Röntgenbilder unterschiedliche Bereiche der Gefäßstruktur mit Kontrastmittel gefüllt sein und/oder die Menge an Kontrastmittel in einem bestimmten Bereich der Gefäßstruktur kann sich für verschiedene der wenigstens zwei Röntgenbilder unterscheiden und/oder eine Gesamtmenge an Kontrastmittel in der Gefäßstruktur kann sich für verschiedene der wenigstens zwei Röntgenbilder unterscheiden.

Die zweiten Aufnahmezeiträume sind insbesondere aufeinanderfolgende Aufnahmezeiträume, die beispielsweise dieselbe Dauer aufweisen können. Jedes der wenigstens zwei Livebilder wird dabei während eines der zweiten Aufnahmezeiträume erzeugt, insbesondere mittels des Röntgenbildgebungssystems. Die wenigstens zweiten Aufnahmezeiträume liegen dabei insbesondere nach den ersten Aufnahmezeiträumen. Beispielsweise werden die wenigstens zwei Livebilder ohne eine Kontrastmittelverabreichung erzeugt. Die Livebilder können beispielsweise während einer Gefäßintervention an der Gefäßstruktur erzeugt werden.

Die Überlagerungsbilder können, insbesondere nacheinander entsprechend der Abfolge der wenigstens zwei Livebilder, auf dem Anzeigegerät angezeigt werden.

Die gemeinsame Gefäßmaske kann beispielsweise einem monochromatischen Bild entsprechen, beispielsweise einem Graustufenbild, bei dem der Grauwert eines Pixels der entsprechenden Kontrastmittelmenge im entsprechenden Bereich der Gefäßstruktur wiedergibt. Es kann sich auch um ein binäres Bild handeln, bei dem Pixel mit einem ersten Binärwert, beispielsweise 1, zu der Gefäßstruktur gehören und Pixel mit einem zweiten Binärwert, beispielsweise 0, nicht zu der Gefäßstruktur gehören oder basierend auf den wenigstens zwei Röntgenbildern nicht der Gefäßstruktur zugeordnet werden konnten. Insbesondere zeigt die Gefäßmaske keine oder möglichst wenige Teile des abzubildenden Bereichs, welche nicht der Gefäßstruktur entsprechen.

Je nach Ausführungsform des Röntgenbildgebungsverfahrens kann die gemeinsame Gefäßmaske genau einem der unterschiedlichen Kontrastmittelfüllzustände entsprechen. Die gemeinsame Gefäßmaske kann jedoch auch einem virtuellen Kontrastmittelfüllzustand entsprechen, der so durch keines der wenigstens zwei Röntgenbilder dargestellt wird.

Die gemeinsame Gefäßmaske kann in verschiedenen Ausführungsformen in unterschiedlicher Weise erzeugt werden. Eine Möglichkeit ist es, eines der wenigstens zwei Röntgenbilder auszuwählen, beispielsweise eines mit einer maximalen Kontrastmittelmenge in der Gefäßstruktur, und daraus, beispielsweise durch bekannte Verfahren zur Gefäßsegmentierung oder durch andere Methoden zur Gefäßextraktion, die beispielsweise auf der Verwendung eines trainierten Maschinenlernmodells basieren, die Gefäßstruktur oder Teile davon extrahiert werden, um die gemeinsame Gefäßmaske zu erzeugen. Es ist auch möglich, basierend auf den wenigstens zwei Röntgenbildern oder daraus extrahierten Darstellungen der Gefäßstruktur, eine Darstellung des virtuellen Kontrastmittelfüllzustands zu erzeugen, beispielsweise unter Verwendung eines trainierten Maschinenlernmodells. Es ist auch möglich, basierend auf den wenigstens zwei Röntgenbildern jeweils eine Darstellung der Gefäßstruktur zu extrahieren und diese zu kombinieren, beispielsweise unter Verwendung eines trainierten Maschinenlernmodells, um die gemeinsame Gefäßmaske zu erzeugen.

Jedes der Überlagerungsbilder wird basierend auf derselben gemeinsamen Gefäßmaske und basierend auf dem entsprechenden Livebild erzeugt. Dies impliziert jedoch nicht notwendigerweise, dass die gemeinsame Gefäßmaske in derselben Weise jedem der Livebilder überlagert wird. Es ist insbesondere auch möglich, dass für jedes Livebild eine Variante der gemeinsamen Gefäßmaske erzeugt wird, beispielsweise durch räumliche Verschiebung der gemeinsamen Gefäßmaske, und diese Variante der gemeinsamen Gefäßmaske dem jeweiligen Livebild überlagert wird.

Indem für jedes Livebild ein individuelles Überlagerungsbild erzeugt wird, können Einflüsse von Bewegungen des Objekts während der Erzeugung der Livebilder wenigstens teilweise kompensiert werden. Da alle Überlagerungsbilder basierend auf derselben gemeinsamen Gefäßmaske erzeugt werden, wird zum einen eine konsistente Darstellung der Gefäßstruktur in allen Überlagerungsbildern erreicht und zum anderen kann der Rechenaufwand insgesamt reduziert werden.

Das Überlagerungsbild kann durch Überlagerung des Livebilds mit der gemeinsamen Gefäßmaske beziehungsweise der jeweiligen Variante der gemeinsamen Gefäßmaske erzeugt werden. Es kann aber auch ein weiteres Subtraktionsbild basierend auf dem Livebild erzeugt werden und das Überlagerungsbild kann durch Überlagerung des weiteren Subtraktionsbilds mit der gemeinsamen Gefäßmaske beziehungsweise der jeweiligen Variante der gemeinsamen Gefäßmaske erzeugt werden. Das weitere Subtraktionsbild kann beispielsweise durch Subtraktion eines weiteren Maskenbilds von dem Livebild erzeugt werden.

Beispielsweise kann das Livebild den abzubildenden Bereich mit einem Werkzeug, etwa einem Katheter, einem Gefäßballon, einem Stent, einem Führungsdraht oder dergleichen, darstellen und das weitere Maskenbild kann den abzubildenden Bereich ohne dem Werkzeug darstellen. In dem weiteren Subtraktionsbild ist daher das Werkzeug gegenüber dem Livebild hervorgehoben. Es kann in diesem Fall beispielsweise von einem Roadmap-Verfahren gesprochen werden, insbesondere wenn die Erzeugung der Subtraktionsbilder vorgesehen ist.

Gemäß zumindest einer Ausführungsform werden wenigstens ein Maskenbild des abzubildenden Bereichs und wenigstens zwei Kontrastmittelbilder des abzubildenden Bereichs erhalten, wobei die wenigstens zwei Kontrastmittelbilder die Gefäßstruktur in den unterschiedlichen Kontrastmittelfüllzuständen darstellen. Die wenigstens zwei Röntgenbilder sind durch die wenigstens zwei Kontrastmittelbilder gegeben.

Dadurch wird der Aufwand zur Erzeugung der wenigstens zwei Röntgenbilder, sowohl der Aufwand zur Erzeugung der Rohdaten mittels eines Röntgenbildgebungssystems sowie der Rechenaufwand zur Erzeugung der wenigstens zwei Röntgenbilder basierend auf den Rohdaten, reduziert.

Gemäß zumindest einer Ausführungsform werden wenigstens ein Maskenbild des abzubildenden Bereichs und wenigstens zwei Kontrastmittelbilder des abzubildenden Bereichs erhalten, wobei die wenigstens zwei Kontrastmittelbilder die Gefäßstruktur in den unterschiedlichen Kontrastmittelfüllzuständen darstellen. Für jedes Kontrastmittelbild der wenigstens zwei Kontrastmittelbilder wird basierend auf dem wenigstens einen Maskenbild ein Subtraktionsbild erzeugt. Die wenigstens zwei Röntgenbilder sind durch die Subtraktionsbilder gegeben.

Die wenigstens zwei Kontrastmittelbilder entsprechen dabei den unterschiedlichen ersten Aufnahmezeiträumen und stellen die Gefäßstruktur in den unterschiedlichen Kontrastmittelfüllzuständen dar.

Dadurch wird erreicht, dass die Gefäßstruktur in den wenigstens zwei Röntgenbildern deutlicher hervorgehoben ist, was letztlich die Genauigkeit der gemeinsamen Gefäßmaske und damit der Überlagerungsbilder erhöht.

Das wenigstens eine Maskenbild kann genau ein Maskenbild sein. Die Subtraktionsbilder können dann beispielsweise durch Subtraktion des Maskenbildes von dem jeweiligen Kontrastmittelbild erzeugt werden. Dadurch wird der Aufwand zur Erzeugung des wenigstens einen Maskenbildes, sowohl der Aufwand zur Erzeugung der Rohdaten mittels des Röntgenbildgebungssystems sowie der Rechenaufwand zur Erzeugung des wenigstens einen Maskenbildes basierend auf den Rohdaten, reduziert.

Das wenigstens eine Maskenbild kann auch zwei oder mehr Maskenbilder beinhalten, beispielsweise ein Maskenbild für jedes der Kontrastmittelbilder. Das jeweilige Subtraktionsbild wird dann durch Subtraktion des zugehörigen Maskenbildes von dem jeweiligen Kontrastmittelbild erzeugt. So kann die Gefäßstruktur in den Subtraktionsbildern genauer dargestellt werden.

Gemäß zumindest einer Ausführungsform enthält das wenigstens eine Maskenbild wenigstens zwei Maskenbilder, wobei die wenigstens zwei Maskenbilder unterschiedlichen weiteren Aufnahmezeiträumen entsprechen. Für jedes der wenigstens zwei Kontrastmittelbilder wird ein entsprechendes Summenbild (9a, 9b) durch gewichtete Summation der wenigstens zwei Maskenbilder erzeugt. Für jedes der wenigstens zwei Kontrastmittelbilder wird das jeweilige Subtraktionsbild durch Subtraktion des jeweiligen Summenbildes (9a, 9b) von dem jeweiligen Kontrastmittelbild erzeugt.

Entsprechende Gewichtungsfaktoren zur Erzeugung des Summenbildes (9a, 9b) können beispielsweise durch ein Optimierungsverfahren automatisch bestimmt werden. Insbesondere kann ein Verfahren verwendet werden, wie es in dem eingangs genannten Dokument DE 10 2021 208 272 A1 beschrieben wird, insbesondere durch Lösung eines Optimierungsproblems, wie in Absatz [0036] des genannten Dokuments definiert.

Dadurch kann die Genauigkeit der Darstellung der Gefäßstruktur in den Subtraktionsbildern erhöht werden, insbesondere kann eine Bewegung des Objekts bei der Erzeugung der Maskenbilder und/oder der Kontrastmittelbilder wenigstens teilweise kompensiert werden. Dies erhöht folglich auch die Genauigkeit der Darstellung der Gefäßstruktur in der gemeinsamen Gefäßmaske beziehungsweise letztlich in den Überlagerungsbildern.

Insbesondere kann die Anzahl der wenigstens zwei Maskenbilder gleich der Anzahl der wenigstens zwei Kontrastmittelbilder sein. Die weiteren Aufnahmezeiträume können beispielsweise vor den ersten Aufnahmezeiträumen liegen, insbesondere vor einer entsprechenden Kontrastmittelverabreichung.

Gemäß zumindest einer Ausführungsform beinhaltet das Erzeugen der gemeinsamen Gefäßmaske das Anwenden wenigstens eines trainierten Maschinenlernmodells, MLM, auf Eingangsdaten, die von den wenigstens zwei Röntgenbildern abhängen, diese beispielsweise beinhalten oder abhängig von diesen erzeugt werden. Die Ausgabe des trainierten Maschinenlernmodells beinhaltet insbesondere die gemeinsame Gefäßmaske oder die gemeinsame Gefäßmaske wird abhängig von der Ausgabe des wenigstens einen trainierten MLM erzeugt.

Allgemein ausgedrückt, kann ein trainiertes MLM kognitive Funktionen nachstellen, die Menschen mit einem anderen menschlichen Verstand in Verbindung bringen. Insbesondere kann das MLM durch Trainieren basierend auf Trainingsdaten in der Lage sein, sich an neue Umstände anzupassen und Muster zu detektieren und zu extrapolieren. Ein anderer Begriff für ein trainiertes MLM ist "trainierte Funktion".

Im Allgemeinen können die Parameter eines MLM durch Trainieren angepasst oder aktualisiert werden. Dabei können insbesondere überwachtes Trainieren, halbüberwachtes Trainieren, unüberwachtes Trainieren, Verstärkungslernen (englisch: reinforcement learning) und/oder aktives Lernen zum Einsatz kommen. Darüber hinaus kann Repräsentationslernen (englisch: representation learning), das auch als Merkmalslernen (englisch: feature learning) bezeichnet wird, verwendet werden. Insbesondere können die Parameter der MLMs iterativ durch mehrere Schritte des Trainierens angepasst werden. Insbesondere kann beim Trainieren eine bestimmte Verlustfunktion, die auch als Kostenfunktion bezeichnet wird, minimiert werden. Beim Trainieren eines künstlichen neuronalen Netzwerks, ANN (englisch: artificial neural network), kann insbesondere der Backpropagation-Algorithmus verwendet werden.

Ein MLM kann insbesondere ein ANN, eine Support-Vector-Maschine, einen Entscheidungsbaum und/oder ein Bayes'sches Netzwerk beinhalten, und/oder das MLM kann auf k-means-Clustering, Q-learning, genetischen Algorithmen und/oder Assoziationsregeln basieren. Insbesondere kann ein ANN ein tiefes neuronales Netzwerk, ein faltendes neuronales Netzwerk, CNN (englisch: convolutional neural network) oder ein faltendes tiefes neuronales Netzwerk sein oder beinhalten. Außerdem kann ein ANN ein adversariales Netzwerk, ein tiefes adversariales Netzwerk und/oder ein generatives adversariales Netzwerk sein.

Gemäß zumindest einer Ausführungsform beinhaltet das Erzeugen der gemeinsamen Gefäßmaske ein Anwenden eines trainierten ersten Maschinenlernmodells auf erste Eingangsdaten, die von den wenigstens zwei Röntgenbildern abhängen.

Das erste MLM kann beispielsweise ein ANN sein, insbesondere ein CNN, beispielsweise ein U-Net oder ein auf einem U-Net basierendes ANN.

Die Ausgabe des trainierten ersten MLM beinhaltet insbesondere die gemeinsame Gefäßmaske oder die gemeinsame Gefäßmaske wird abhängig von der Ausgabe des trainierten ersten MLM erzeugt.

Die Eingangsdaten können in manchen Ausführungsformen die wenigstens zwei Röntgenbildern enthalten, insbesondere wenn das Erzeugen der Subtraktionsbilder nicht vorgesehen ist.

In Ausführungsformen, die das Erzeugen der Subtraktionsbilder vorsehen, können die ersten Eingangsdaten beispielsweise von den Subtraktionsbildern abhängen, diese insbesondere beinhalten. Alternativ kann für jedes der Subtraktionsbilder eine Kombination des jeweiligen Subtraktionsbilds mit dem zugehörigen Kontrastmittelbild erzeugt werden, beispielsweise durch gewichtete Summation. In der resultierenden Kombination ist die Gefäßstruktur gegenüber dem jeweiligen Kontrastmittelbild insbesondere hervorgehoben. Die ersten Eingangsdaten können beispielsweise von den Kombinationen abhängen, diese insbesondere beinhalten.

Das erste MLM kann beispielsweise dazu trainiert sein, basierend auf allen Röntgenbildern der wenigstens zwei Röntgenbilder die gemeinsame Gefäßmaske zu prädizieren. Dabei kann die gemeinsame Gefäßmaske beispielsweise die aus einem der Röntgenbilder extrahierte Gefäßstruktur darstellen, beispielsweise aus derjenigen mit einer optimalen, insbesondere maximalen, Füllung der Gefäßstruktur mit dem Kontrastmittel. Alternativ kann die gemeinsame Gefäßmaske beispielsweise die Gefäßstruktur in dem virtuellen Kontrastmittelfüllzustand darstellen, welche das erste MLM basierend auf allen der wenigstens zwei Röntgenbilder prädiziert.

Für jedes Röntgenbild der wenigstens zwei Röntgenbilder wird eine vorläufige Gefäßmaske erzeugt, indem ein trainiertes erstes MLM auf erste Eingangsdaten angewendet wird, welche von dem jeweiligen Röntgenbild abhängen. Die gemeinsame Gefäßmaske wird abhängig von den vorläufigen Gefäßmasken erzeugt.

Die vorläufigen Gefäßmasken können beispielsweise die Gefäßstruktur in dem Kontrastmittelfüllzustand des jeweiligen Röntgenbilds darstellen. Das erste MLM ist also insbesondere dazu trainiert, die Gefäßstruktur aus dem jeweiligen Röntgenbild zu extrahieren, insbesondere wenn das Erzeugen der Subtraktionsbilder nicht vorgesehen ist. In Ausführungsformen, die das Erzeugen der Subtraktionsbilder vorsehen, können die ersten Eingangsdaten beispielsweise von den Subtraktionsbildern abhängen, diese insbesondere beinhalten. Das erste MLM ist dann insbesondere dazu trainiert, die Gefäßstruktur aus dem jeweiligen Subtraktionsbild zu extrahieren. Alternativ kann für jedes der Subtraktionsbilder eine Kombination des jeweiligen Subtraktionsbilds mit dem zugehörigen Kontrastmittelbild erzeugt werden, beispielsweise durch gewichtete Summation. In der resultierenden Kombination ist die Gefäßstruktur gegenüber dem jeweiligen Kontrastmittelbild insbesondere hervorgehoben. Die ersten Eingangsdaten können beispielsweise von den Kombinationen abhängen, diese insbesondere beinhalten. Das erste MLM ist dann insbesondere dazu trainiert, die Gefäßstruktur aus der jeweiligen Kombination zu extrahieren.

Die Verwendung des ersten MLM hat beispielsweise den Vorteil, dass das erste MLM universell eingesetzt werden kann und die Gefäßstruktur mit hoher Genauigkeit extrahieren kann.

Alternativ zu dem ersten MLM kann in manchen Ausführungsformen auch ein anderes, insbesondere an sich bekanntes, Verfahren zur Gefäßextraktion oder Gefäßsegmentierung, insbesondere zum Erzeugen der vorläufigen Gefäßmasken, verwendet werden. Dadurch kann insbesondere der Aufwand zum Trainieren eines MLM eingespart werden.

Gemäß zumindest einer Ausführungsform wird die gemeinsame Gefäßmaske gemäß einer vorgegebenen Regel als eine der vorläufigen Gefäßmasken ausgewählt.

Die vorgegebene Regel kann beispielsweise festlegen, dass die nach vorgegebenen Kriterien beste der vorläufigen Gefäßmasken ausgewählt wird. Die beste vorläufige Gefäßmaske kann dabei beispielsweise diejenige sein, die dem Kontrastmittelfüllzustand mit der größten Gesamtmenge an Kontrastmittel entspricht. Die Gesamtmenge an Kontrastmittel kann beispielsweise durch Berechnung einer L2-Norm der jeweiligen vorläufigen Gefäßmaske oder einer sonstigen geeigneten Metrik bestimmt werden.

Dadurch wird erreicht, dass für jedes Überlagerungsbild der Kontrastmittelfüllzustand mit der größten Gesamtmenge an Kontrastmittel dargestellt wird. Dem medizinischen Personal kann also die maximal verfügbare Information bereitgestellt werden.

Gemäß zumindest einer Ausführungsform wird die gemeinsame Gefäßmaske durch Anwendung eines trainierten zweiten MLM auf zweite Eingangsdaten, welche von den vorläufigen Gefäßmasken abhängen, diese insbesondere beinhalten, erzeugt.

Das zweite MLM kann dazu trainiert sein, die vorläufige Gefäßmaske gemäß der vorgegebenen Regel als die gemeinsame Gefäßmaske zu prädizieren. Hierdurch können insbesondere komplexere Regeln realisiert werden, beispielsweise betreffend die räumliche Verteilung des Kontrastmittels in der Gefäßstruktur oder die ausreichende Füllung bestimmter kritischer Teile der Gefäßstruktur mit dem Kontrastmittel.

Das zweite MLM kann auch dazu trainiert sein, die gemeinsame Gefäßmaske derart aus den vorläufigen Gefäßmasken zu prädizieren, dass sie den virtuellen Kontrastmittelfüllzustand darstellt. Dadurch kann die dem medizinischen Personal bereitgestellte Information weiter verbessert werden.

Gemäß zumindest einer Ausführungsform wird abhängig von der gemeinsamen Gefäßmaske für jede vorläufige Gefäßmaske der vorläufigen Gefäßmasken eine Registrierungsvorschrift ermittelt. Für jedes der wenigstens zwei Livebilder wird durch Abgleich des jeweiligen Livebilds mit den wenigstens zwei Röntgenbildern eines der wenigstens zwei Röntgenbildern identifiziert. Für jedes der wenigstens zwei Livebilder wird zum Erzeugen des Überlagerungsbildes die Gefäßmaske anhand derjenigen Registrierungsvorschrift, welche dem identifizierten Röntgenbild entspricht, zu dem jeweiligen Livebild registriert und dem jeweiligen Livebild überlagert.

Die Registrierungsvorschrift kann beispielsweise einen Verschiebungsvektor beinhalten. Werden die Pixel der gemeinsamen Gefäßmaske um den Verschiebungsvektor verschoben, so resultiert eine verschobene Gefäßmaske, die zumindest teilweise näherungsweise mit der jeweiligen vorläufigen Gefäßmaske übereinstimmt. Die Registrierungsvorschrift kann beispielsweise für jedes Pixel der gemeinsamen Gefäßmaske oder für jede Pixelgruppe einer Vielzahl vordefinierter Pixelgruppen der gemeinsamen Gefäßmaske einen entsprechenden Verschiebungsvektor beinhalten.

Die Registrierungsvorschrift kann beispielsweise durch ein Optimierungsverfahren bestimmt werden, mit dem eine Metrik zur Quantifizierung einer Abweichung zwischen der gemeinsamen Gefäßmaske und der jeweiligen vorläufigen Gefäßmaske minimiert wird oder eine Metrik zur Quantifizierung einer Übereinstimmung zwischen der gemeinsamen Gefäßmaske und der jeweiligen vorläufigen Gefäßmaske maximiert wird.

Beispielsweise kann die Registrierungsvorschrift durch Maximierung einer normalisierten Kreuzkorrelation, NCC (englisch: normalized cross correlation), zwischen der gemeinsamen Gefäßmaske und der jeweiligen vorläufigen Gefäßmaske bestimmt werden. Alternativ dazu kann eine auf Gradientenoptimierung basierende Registrierungstechnik verwendet werden oder es kann ein weiteres trainiertes MLM zur Registrierung verwendet werden.

Bezeichnet man die wenigstens zwei Röntgenbilder mit Rᵢ, wobei i = 1, ..., N mit N ≥ 2 die einzelnen Röntgenbilder indexiert, so können die vorläufigen Gefäßmasken mit VMᵢ bezeichnet werden und die zugehörigen Registrierungsvor-schriften mit Pᵢ. Jedem i = 1, ..., N sind also ein VMᵢ, ein Rᵢ und ein Pᵢ zugeordnet. Analog können die wenigstens zwei Livebilder mit Lₖ bezeichnet werden, wobei k = 1, ..., M mit M ≥ 2.

Ein gegebenes Livebild Lₖ wird dann insbesondere mit allen Röntgenbildern Rᵢ abgeglichen, insbesondere durch Berechnung einer geeigneten Metrik, welche die Ähnlichkeit zwischen Lₖ und Rᵢ angibt, zum Beispiel die NCC. Dasjenige Rᵢ mit der besten Übereinstimmung mit dem gegebenen Lₖ wird identifiziert, also beispielsweise dasjenige mit der größten Metrik, beispielsweise L2-Norm. Das zugehörige i kann als i* bezeichnet werden.

Zum Erzeugen des Überlagerungsbildes wird dann die gemeinsame Gefäßmaske pixelweise entsprechend der Registrierungs-vorschrift P_{i*} verschoben, was in einer angepassten oder verschobenen Gefäßmaske resultiert. Die verschobene Gefäßmaske wird dann dem Livebild Lₖ überlagert, um das entsprechende Überlagerungsbild zu erzeugen.

Dies kann für alle k = 1, ..., M analog durchgeführt werden. In alternativen Ausführungsformen wird das Livebild mit den Kontrastmittelbildern abgeglichen, um i* zu bestimmen, wie weiter unten erläutert wird.

Wie bereits erwähnt, kann es sich bei den wenigstens zwei Röntgenbildern um die wenigstens zwei Subtraktionsbilder handeln. Auch in diesem Fall wird den Livebildern jedoch nicht das am besten zum Livebild passende Subtraktionsbild überlagert, sondern die wie beschrieben verschobene gemeinsame Gefäßmaske. Die hat insbesondere den Vorteil, dass so unabhängig vom aktuellen Bewegungszustand, beispielsweise in einem Atemzyklus, stets dieselbe gemeinsame Gefäßmaske zugrunde gelegt wird, die insbesondere den Kontrastmittelfüllzustand mit der größten Gesamtmenge an Kontrastmittel darstellt oder den virtuellen Kontrastmittelfüllzustand. So kann für mehrere aufeinanderfolgende Livebilder auch bei Bewegung während deren Aufnahme stets ein optimales Überlagerungsbild erzeugt werden.

Gemäß zumindest einer Ausführungsform wird abhängig von der gemeinsamen Gefäßmaske für jede der vorläufigen Gefäßmasken die Registrierungsvorschrift ermittelt. Für jedes der wenigstens zwei Livebilder wird durch Abgleich des jeweiligen Livebilds mit den wenigstens zwei Kontrastmittelbildern eines der Kontrastmittelbilder identifiziert. Für jedes der wenigstens zwei Livebilder wird zum Erzeugen des Überlagerungsbildes die gemeinsame Gefäßmaske anhand derjenigen Registrierungsvorschrift, welche dem identifizierten Kontrastmittelbild entspricht, zu dem jeweiligen Livebild registriert und dem jeweiligen Livebild überlagert.

Die obigen Erläuterungen zu Ausführungsformen, bei denen der Abgleich des Livebilds mit den Röntgenbildern vorgesehen ist, können analog übertragen werden.

Insbesondere wenn das Erzeugen der Subtraktionsbilder vorgesehen ist, kann der Abgleich der Livebilder mit den Kontrastmittelbildern gegenüber einem Abgleich der Livebilder mit den Subtraktionsbildern vorteilhaft sein, da die Kontrastmittelbilder ebenso wie die Livebilder nicht nur die Gefäßstruktur darstellen, sondern auch den anatomischen Hintergrund.

Gemäß zumindest einer Ausführungsform beinhaltet das erste MLM ein CNN, beispielsweise ein U-Net oder ein basierend auf dem U-Net entworfenes CNN, oder ein Transformernetzwerk oder ein TransUNet oder ein basierend auf dem TransUNet entworfenes ANN oder ein MTM oder ein basierend auf dem MTM entworfenes ANN.

Gemäß zumindest einer Ausführungsform werden die wenigstens zwei Röntgenbilder während einer Kontrastmittelverabreichung in die Gefäßstruktur erzeugt.

So lassen sich die Röntgenbilder zu unterschiedlichen Kontrastmittelfüllzuständen erzeugen.

Gemäß zumindest einer Ausführungsform werden die wenigstens zwei Kontrastmittelbilder während der Kontrastmittelverabreichung in die Gefäßstruktur erzeugt.

Gemäß zumindest einer Ausführungsform stellen die wenigstens zwei Röntgenbilder die Gefäßstruktur während unterschiedlicher Bewegungszustände des Objekts dar.

Die unterschiedlichen Bewegungszustände können beispielsweise unterschiedlichen Bewegungszuständen einer zyklischen Bewegung, etwa einer Atembewegung oder einer Herzbewegung, entsprechen. Es kann sich aber auch um andere, insbesondere nicht-zyklische Bewegungen des Objekts handeln.

Gemäß zumindest einer Ausführungsform stellt wenigstens ein Livebild der wenigstens zwei Livebilder ein Werkzeug zur Gefäßintervention in dem abzubildenden Bereich dar.

Gemäß einem weiteren Aspekt der Erfindung wird eine Datenverarbeitungsvorrichtung angegeben. Die Datenverarbeitungsvorrichtung weist wenigstens eine Recheneinheit auf, die angepasst ist, ein erfindungsgemäßes Röntgenbildgebungsverfahren durchzuführen.

In der vorliegenden Offenbarung können die Ausdrücke "Datenverarbeitungsvorrichtung" und "wenigstens eine Recheneinheit" austauschbar verwendet werden. Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application-specific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß einem weiteren Aspekt der Erfindung wird ein Röntgenbildgebungssystem angegeben. Das Röntgenbildgebungssystem weist eine erfindungsgemäße Datenverarbeitungsvorrichtung auf. Das Röntgenbildgebungssystem weist eine Röntgenquelle und einen Röntgendetektor zum Erzeugen der wenigstens zwei Livebilder und/oder der wenigstens zwei Röntgenbilder und/oder des wenigstens einen Maskenbildes und/oder der wenigstens zwei Kontrastmittelbilder auf.

Gemäß zumindest einer Ausführungsform weist das Röntgenbildgebungssystem das Anzeigegerät auf und die wenigstens eine Recheneinheit ist dazu eingerichtet, die Überlagerungsbilder auf dem Anzeigegerät anzuzeigen.

Weitere Ausführungsformen des erfindungsgemäßen Röntgenbildgebungssystems folgen unmittelbar aus den verschiedenen Ausgestaltungen des erfindungsgemäßen Röntgenbildgebungsverfahrens und umgekehrt. Insbesondere lassen sich einzelne Merkmale und entsprechende Erläuterungen sowie Vorteile bezüglich der verschiedenen Ausführungsformen zu dem erfindungsgemäßen Röntgenbildgebungsverfahren analog auf entsprechende Ausführungsformen des erfindungsgemäßen Röntgenbildgebungssystems übertragen.

Gemäß einem weiteren Aspekt der Erfindung wird ein Computerprogramm mit Befehlen angegeben. Wenn die Befehle durch wenigstens eine Recheneinheit ausgeführt werden, veranlassen die Befehle die wenigstens eine Recheneinheit dazu, ein erfindungsgemäßes Röntgenbildgebungsverfahren durchzuführen.

Die Befehle können beispielsweise als Programmcode vorliegen. Der Programmcode kann beispielsweise als Binärcode oder Assembler und/oder als Quellcode einer Programmiersprache, zum Beispiel C**,** und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm speichert.

Das Computerprogramm und das computerlesbare Speichermedium sind jeweils Computerprogrammprodukte mit den Befehlen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer beispielhaften Ausführungsform eines erfindungsgemäßen Röntgenbildgebungssystems;
- FIG 2: ein Ablaufdiagramm einer beispielhaften Ausführungsform eines erfindungsgemäßen Röntgenbildgebungsverfahrens;
- FIG 3: ein Ablaufdiagramm einer weiteren beispielhaften Ausführungsform eines erfindungsgemäßen Röntgenbildgebungsverfahrens;
- FIG 4: ein Ablaufdiagramm einer weiteren beispielhaften Ausführungsform eines erfindungsgemäßen Röntgenbildgebungsverfahrens;
- FIG 5: schematisch eine beispielhafte Ausführungsform eines faltenden neuronalen Netzwerks; und
- FIG 6: schematisch eine beispielhafte Ausführungsform eines faltenden neuronales Netzwerks mit einer U-Net-Struktur.

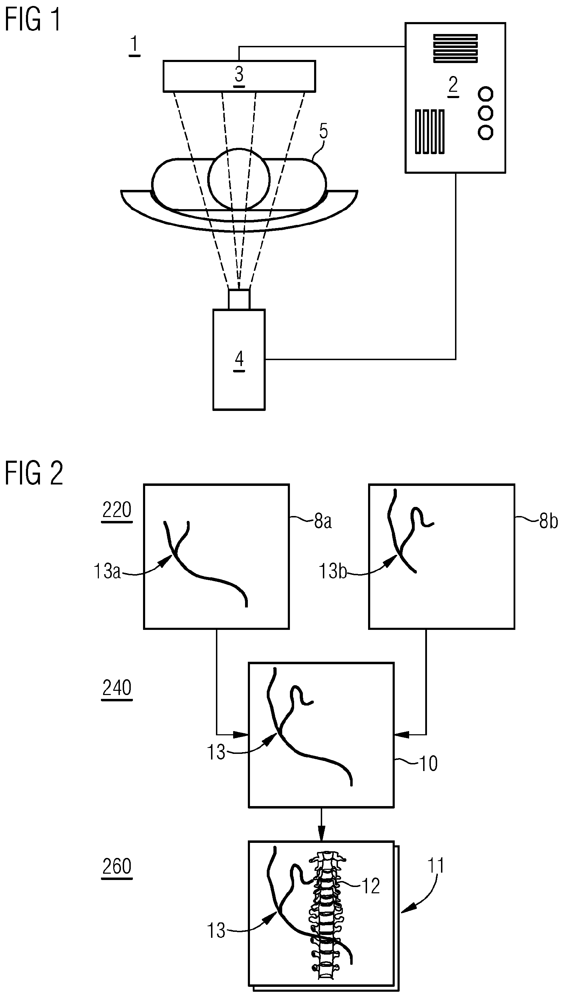

In FIG 1 ist schematisch eine beispielhaften Ausführungsform eines erfindungsgemäßen Röntgenbildgebungssystems 1 dargestellt. Das Röntgenbildgebungssystem 1 weist eine Röntgenquelle 4 und einen Röntgendetektor 3 auf, sowie wenigstens eine Recheneinheit 2, die dazu eingerichtet ist, ein erfindungsgemäßes Röntgenbildgebungsverfahren durchzuführen.

FIG 2 zeigt ein Ablaufdiagramm einer beispielhaften Ausführungsform eines erfindungsgemäßen Röntgenbildgebungsverfahrens.

In Schritt 220 werden wenigstens zwei Röntgenbilder 8a, 8b eines abzubildenden Bereichs eines Objekts 5 erhalten. Die wenigstens zwei Röntgenbilder 8a, 8b entsprechen unterschiedlichen ersten Aufnahmezeiträumen und stellen eine Gefäßstruktur 13, 13a, 13b des Objekts 5 in unterschiedlichen Kontrastmittelfüllzuständen dar. Die Röntgenbilder 8a, 8b werden beispielsweise mittels des Röntgenbildgebungssystems 1 während einer Kontrastmittelverabreichung in die Gefäßstruktur 13, 13a, 13b erzeugt und entsprechen beispielsweise unterschiedlichen Bewegungszuständen des Objekts 5.

In Schritt 240 wird basierend auf den wenigstens zwei Röntgenbildern 8a, 8b eine gemeinsame Gefäßmaske 10 erzeugt, welche die Gefäßstruktur 13, 13a, 13b darstellt. In Schritt 260 werden wenigstens zwei Livebilder des abzubildenden Bereichs, welche unterschiedlichen zweiten Aufnahmezeiträumen entsprechen, erhalten. Im Schritt 260 wird auch für jedes der wenigstens zwei Livebilder abhängig von der gemeinsamen Gefäßmaske 10 ein entsprechendes Überlagerungsbild 11 zur Anzeige auf einem Anzeigegerät des Röntgenbildgebungssystems 1 erzeugt und beispielsweise auf dem Anzeigegerät angezeigt.

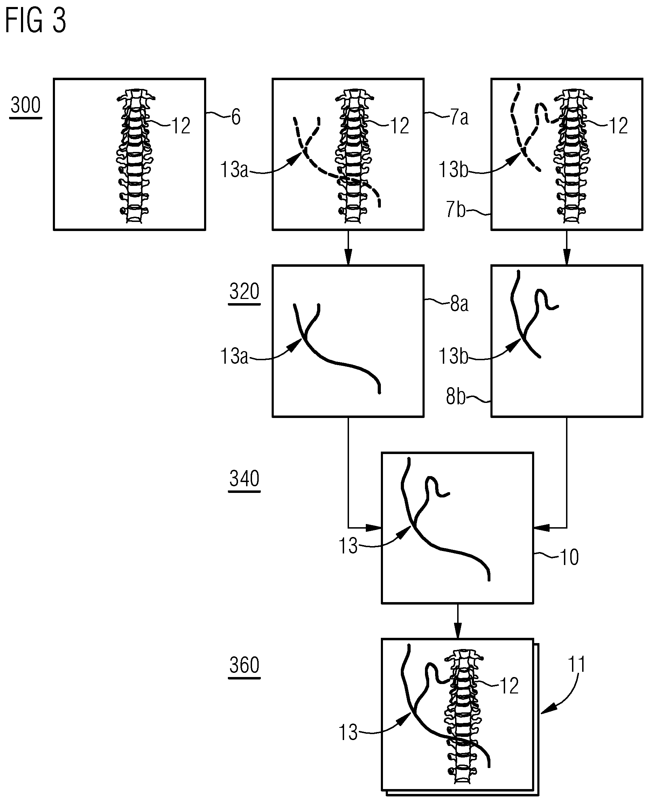

FIG 3 zeigt ein Ablaufdiagramm einer weiteren beispielhaften Ausführungsform eines erfindungsgemäßen Röntgenbildgebungsverfahrens.

Hierbei werden die wenigstens zwei Röntgenbilder 8a, 8b als Subtraktionsbilder 8a, 8b, insbesondere im Rahmen einer DSA, erzeugt.

In Schritt 300 wird wenigstens ein Maskenbild 6 des abzubildenden Bereichs erhalten, und es werden wenigstens zwei Kontrastmittelbilder 7a, 7b des abzubildenden Bereichs erhalten, wobei die wenigstens zwei Kontrastmittelbilder 7a, 7b die Gefäßstruktur 13, 13a, 13b in den unterschiedlichen Kontrastmittelfüllzuständen darstellen.

In Schritt 320 wird für jedes der wenigstens zwei Kontrastmittelbilder 7a, 7b basierend auf dem wenigstens einen Maskenbild 6 ein Subtraktionsbild 8a, 8b erzeugt. Beispielweise wird von jedem der wenigstens zwei Kontrastmittelbilder 7a, 7b das Maskenbild 6, welches ohne Kontrastmittelgabe erzeugt wird, subtrahiert um ein entsprechendes Subtraktionsbild 8a, 8b zu erzeugen. Das Maskenbild 6 zeigt beispielsweise einen anatomischen Hintergrund 12 sowie die Gefäßstruktur 13, 13a, 13b, wobei die Gefäßstruktur 13, 13a, 13b im Maskenbild 6 in FIG 3 nicht dargestellt ist. In den Subtraktionsbildern 8a, 8b ist der anatomische Hintergrund 12 beispielsweise unterdrückt.

Alternativ werden in Schritt 300 wenigstens zwei Maskenbilder 6 erhalten, wobei die wenigstens zwei Maskenbilder 6 unterschiedlichen weiteren Aufnahmezeiträumen entsprechen. Die Subtraktionsbilder 8a, 8b werden basierend auf den wenigstens zwei Maskenbildern 6 und den wenigstens zwei Kontrastmittelbildern 7a, 7b erzeugt.

Dabei wird beispielsweise für jedes der wenigstens zwei Kontrastmittelbilder 7a, 7b ein entsprechendes Summenbild (9a, 9b) durch gewichtete Summation der wenigstens zwei Maskenbilder 6 erzeugt. Für jedes der wenigstens zwei Kontrastmittelbilder 7a, 7b wird das jeweilige Subtraktionsbild 8a, 8b durch Subtraktion des jeweiligen Summenbildes (9a, 9b) von dem jeweiligen Kontrastmittelbild 7a, 7b erzeugt.

In Schritt 340 wird basierend auf den wenigstens zwei Subtraktionsbildern 8a, 8b eine gemeinsame Gefäßmaske 10 erzeugt, welche die Gefäßstruktur 13, 13a, 13b darstellt. Dazu wird beispielsweise ein entsprechend trainiertes MLM auf Eingangsdaten angewendet, welche die Subtraktionsbilder 8a, 8b beinhalten.

In Schritt 360 werden wenigstens zwei Livebilder des abzubildenden Bereichs, welche unterschiedlichen zweiten Aufnahmezeiträumen entsprechen, erhalten. Im Schritt 360 wird auch für jedes der wenigstens zwei Livebilder abhängig von der gemeinsamen Gefäßmaske 10 ein entsprechendes Überlagerungsbild 11 zur Anzeige auf einem Anzeigegerät des Röntgenbildgebungssystems 1 erzeugt und beispielsweise auf dem Anzeigegerät angezeigt.

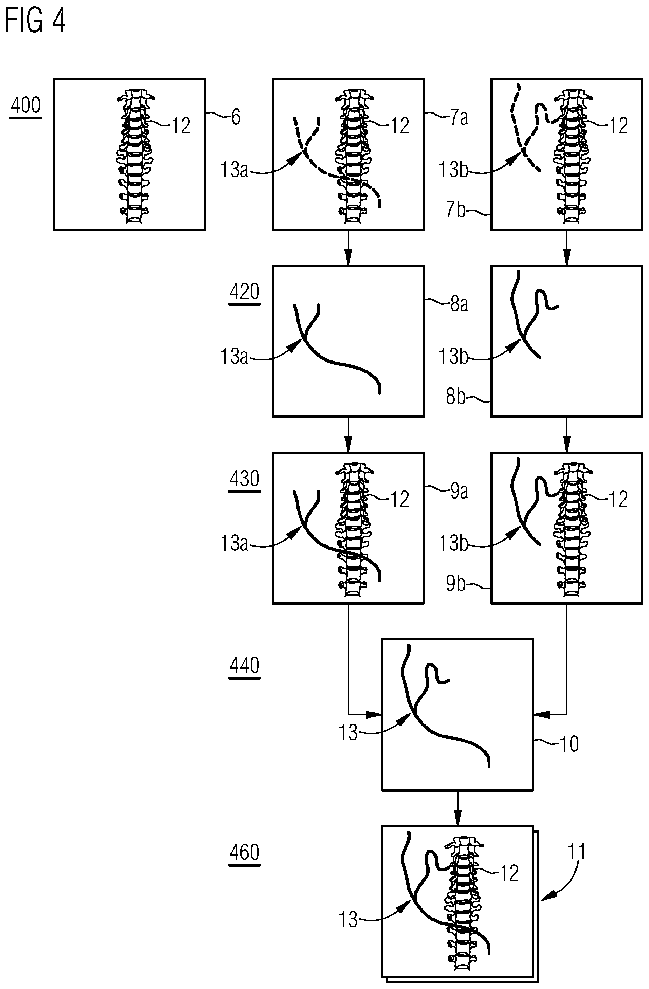

FIG 4 zeigt ein Ablaufdiagramm einer weiteren beispielhaften Ausführungsform eines erfindungsgemäßen Röntgenbildgebungsverfahrens, welches auf der Ausführungsform der FIG 3 basiert, wobei wenigstens zwei Maskenbilder 6 erhalten werden. Die Schritte 400, 420 und 460 entsprechen den Schritten 300, 320 beziehungsweise 360. Die Erzeugung der gemeinsamen Gefäßmaske 10 unterscheidet sich in dem Verfahren gemäß FIG 4 von dem Verfahren gemäß FIG 3.

Im optionalen Schritt 430 wird für jedes der wenigstens zwei Subtraktionsbilder 8a, 8b eine Kombination durch gewichtete Summation der wenigstens zwei Maskenbilder 6 ein entsprechendes Summenbild 9a, 9b erzeugt. Die Summenbilder 9a, 9b können dabei als modifizierte Kontrastmittelbilder 7a, 7b aufgefasst werden, in denen die Gefäßstruktur 13, 13a, 13b jeweils hervorgehoben ist. Es wird für jedes der wenigstens zwei Kontrastmittelbilder 7a, 7b das jeweilige Subtraktionsbild 8a, 8b durch Subtraktion des jeweiligen Summenbildes 9a, 9b von dem jeweiligen Kontrastmittelbild 7a, 7b erzeugt.

Für jedes der wenigstens zwei Subtraktionsbilder 8a, 8b wird eine vorläufige Gefäßmaske erzeugt, indem ein trainiertes weiteres MLM auf weitere Eingangsdaten, welche das jeweilige Subtraktionsbild 8a, 8b enthält, angewendet wird. Alternativ wird für jedes der wenigstens zwei Summenbilder 9a, 9b eine vorläufige Gefäßmaske erzeugt, indem das trainierte weitere MLM auf weitere Eingangsdaten, welche das jeweilige Summenbild 9a, 9b enthält, angewendet wird.

Das weitere MLM ist insbesondere zur Gefäßextraktion oder Gefäßsegmentierung trainiert. Die vorläufigen Gefäßmasken stellen also insbesondere die aus dem jeweiligen Subtraktionsbild 8a, 8b oder Summenbild 9a, 9b extrahierte Gefäßstruktur 13, 13a, 13b dar. Das weitere MLM kann beispielsweise ein CNN, insbesondere ein U-Net, zur Gefäßextraktion sein.

Die gemeinsame Gefäßmaske 10 wird in Schritt 440 gemäß einer vorgegebenen Regel als eine der vorläufigen Gefäßmasken ausgewählt. Beispielsweise wird dazu diejenige vorläufige Gefäßmaske ausgewählt welche die größte Pixel-L2-Norm hat, also diejenige, die den Kontrastmittelfüllzustand mit der größten Kontrastmittelmenge darstellt. Alternativ dazu können auch andere Bildmetriken oder auf maschinellem Lernen basierende Verfahren für die Auswahl verwendet werden und/oder es können vorläufige Gefäßmasken kombiniert werden, um die gemeinsame Gefäßmaske 10 zu erzeugen.

In Schritt 460 werden wenigstens zwei Livebilder des abzubildenden Bereichs, welche unterschiedlichen zweiten Aufnahmezeiträumen entsprechen, erhalten. Im Schritt 460 wird auch für jedes der wenigstens zwei Livebilder abhängig von der gemeinsamen Gefäßmaske 10 ein entsprechendes Überlagerungsbild 11 zur Anzeige auf einem Anzeigegerät des Röntgenbildgebungssystems 1 erzeugt und beispielsweise auf dem Anzeigegerät angezeigt.

In manchen Ausführungsformen wird abhängig von der gemeinsamen Gefäßmaske 10 für jede der vorläufigen Gefäßmasken eine Registrierungsvorschrift ermittelt, beispielsweise, indem ein Verschiebungsvektor zwischen der gemeinsamen Gefäßmaske 10 und der jeweiligen vorläufigen Gefäßmaske ermittelt wird, durch den die NCC zwischen der gemeinsamen Gefäßmaske 10 und der jeweiligen vorläufigen Gefäßmaske maximiert wird.

Dazu wird beispielsweise eine erschöpfende Suche (englisch: exhaustive search) auf einer Bildpyramide mit mehreren Auflösungen innerhalb des typischen Bereichs der Bewegung des Objekts 5 durchgeführt. Alternativ können auch auf Gradientenoptimierung basierende oder auf maschinellem Lernen basierende Registrierungstechniken verwendet werden. Dementsprechend wird die gemeinsame Gefäßmaske 10 durch pixelweises Verschieben unter Verwendung des gefundenen optimalen Verschiebungsvektors berechnet.

Beispielsweise wird für jedes der wenigstens zwei Livebilder ein Abgleich des jeweiligen Livebilds mit den wenigstens zwei Kontrastmittelbildern 7a, 7b durchgeführt, um eines der Kontrastmittelbilder 7a, 7b zu identifizieren. Dies wird beispielsweise durchgeführt, indem die NCC zwischen dem jeweiligen Livebild und dem jeweiligen Kontrastmittelbild 7a, 7b berechnet wird, und dasjenige Kontrastmittelbild 7a, 7b identifiziert wird, welche mit dem jeweiligen Livebild die größte NCC aufweist.

Beispielsweise wird zum Erzeugen des jeweiligen Überlagerungsbildes 11 die gemeinsame Gefäßmaske 10 anhand derjenigen Registrierungsvorschrift, welche dem identifizierten Kontrastmittelbild 7a, 7b entspricht, zu dem jeweiligen Livebild registriert und dem jeweiligen Livebild überlagert.

In verschiedenen Ausführungsformen des erfindungsgemäßen Röntgenbildgebungsverfahrens wird für jedes Livebild, insbesondere jeden Bewegungszustand, beispielsweise jede Atemphase, eine Gefäßmaske mit ausreichender Kontrastmittelfüllung zur Verfügung gestellt. Ergänzend kann gegebenenfalls eine angepasste Aufnahme mit verlängerter Kontrastmittelverabreichung durchgeführt werden.

In verschiedenen Ausführungsformen ergibt sich der Vorteil, dass die mittels eines MLM erhaltenen Gefäßmasken kein Rauschen und weniger anatomische Artefakte aufweisen als die Subtraktionsbilder, was zu einer verbesserten Bildqualität in den Überlagerungsbildern führt.

In verschiedenen Ausführungsformen wird die Registrierung nur bezüglich der vorläufigen Gefäßmasken durchgeführt, die keine Knochenstrukturen enthalten und unabhängig von der Bewegung sind. Dadurch kann die Genauigkeit von bekannten Bewegungskompensationsfunktionen verbessert werden.

In verschiedenen Ausführungsformen können die verschobenen Gefäßmasken innerhalb weniger Sekunden erzeugt werden und die Überlagerungsbilder in Echtzeit berechnet werden. Der aufwändigste Rechenschritt ist beispielsweise die Registrierung, der jedoch mit Hilfe von GPUs leicht parallelisiert werden kann.

Ein MLM wie es in manchen Ausführungsformen eingesetzt wird kann zum Beispiel ein ANN sein, insbesondere ein CNN.

Ein CNN ist ein ANN, das in mindestens einer seiner Schichten eine Faltungsoperation anstelle einer allgemeinen Matrixmultiplikation verwendet. Diese Schichten werden als Faltungsschichten bezeichnet. Insbesondere führt eine Faltungsschicht ein Punktprodukt von einem oder mehreren Faltungskernen mit den Eingabedaten der Faltungsschicht durch, wobei die Einträge des einen oder der mehreren Faltungskerne Parameter oder Gewichte sind, die durch Training angepasst werden können. Insbesondere kann man das innere Frobenius-Produkt und die ReLU-Aktivierungsfunktion verwenden. Ein faltendes neuronales Netzwerk kann zusätzliche Schichten umfassen, beispielsweise Pooling-Schichten, vollständig verbundene Schichten und/oder Normalisierungsschichten.

Durch die Verwendung von faltenden neuronalen Netzwerken kann die Eingabe sehr effizient verarbeitet werden, da eine Faltungsoperation, die auf verschiedenen Kerneln basiert, verschiedene Bildmerkmale extrahieren kann, so dass durch die Anpassung der Gewichte des Faltungskernels die relevanten Bildmerkmale während des Trainings bestimmt werden können. Außerdem müssen aufgrund der gemeinsamen Nutzung der Gewichte in den Faltungskernen weniger Parameter trainiert werden, was eine Überanpassung in der Trainingsphase verhindert und ein schnelleres Training oder mehr Schichten im Netzwerk ermöglicht, wodurch die Leistung des Netzwerks verbessert wird.

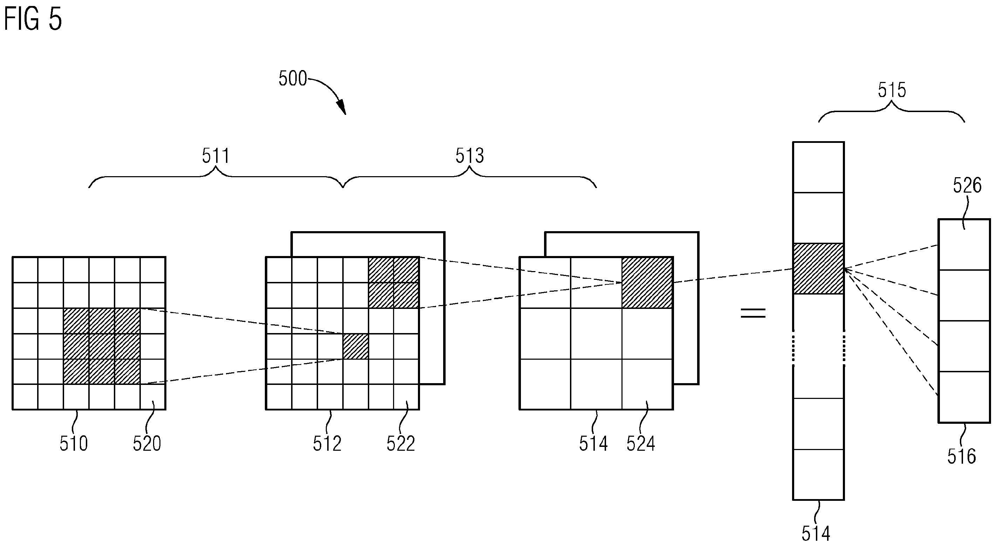

FIG 5 zeigt eine beispielhafte Ausführungsform eines faltenden neuronalen Netzwerks 500. In der dargestellten Ausführungsform umfasst das faltende neuronale Netzwerk 500 eine Eingangsknotenschicht 510, eine Faltungsschicht 511, eine Pooling-Schicht 513, eine vollständig verbundene Schicht 514 und eine Ausgangsknotenschicht 516 sowie verborgene Knotenschichten 512, 514. Alternativ kann das faltende neuronale Netzwerk 500 auch mehrere Faltungsschichten 511, mehrere Pooling-Schichten 513 und/oder mehrere vollständig verbundene Schichten 515 sowie andere Arten von Schichten umfassen. Die Reihenfolge der Schichten kann beliebig gewählt werden, in der Regel werden als letzte Schichten vor der Ausgabeschicht 516vollständig verbundene Schichten 515 verwendet.

Insbesondere können in einem faltenden neuronalen Netzwerk 500 die Knoten 520, 522, 524 einer Knotenschicht 510, 512, 514 als eine d-dimensionale Matrix oder als ein d-dimensionales Bild betrachtet werden. Insbesondere kann im zweidimensionalen Fall der Wert des mit i und j indizierten Knotens 520, 522, 524 in der n-ten Knotenschicht 510, 512, 514 als x(n)[i, j] bezeichnet werden. Die Anordnung der Knoten 520, 522, 524 einer Knotenschicht 510, 512, 514 hat jedoch als solches keinen Einfluss auf die Berechnungen, die innerhalb des faltenden neuronalen Netzwerks 500 durchgeführt werden, da diese allein durch die Struktur und die Gewichte der Kanten gegeben sind.

Eine Faltungsschicht 511 ist eine Verbindungsschicht zwischen einer vorderen Knotenschicht 510 mit Knotenwerten x(n-1) und einer hinteren Knotenschicht 512 mit Knotenwerten x(n). Eine Faltungsschicht 511 ist insbesondere durch die Struktur und die Gewichte der eingehenden Kanten gekennzeichnet, die eine Faltungsoperation auf der Grundlage einer bestimmten Anzahl von Kernen bilden. Insbesondere sind die Struktur und die Gewichte der Kanten der Faltungsschicht 511 so gewählt, dass die Werte x(n) der Knoten 522 der hinteren Knotenschicht 512 als eine Faltung x(n) = K * x(n-1) auf der Grundlage der Werte x(n-1) der Knoten 520 der vorderen Knotenschicht 510 berechnet werden, wobei die Faltung * im zweidimensionalen Fall definiert ist als ${x}^{\left(n\right)} \left[i, j\right] = \left(K∗{x}^{\left(n-1\right)}\right) \left[i, j\right] = {\sum }_{i ′} {\sum }_{j ′} K \left[i′,j′\right] ⋅ {x}^{\left(n-1\right)} \left[i-i′,j-j′\right] .$

Dabei ist der Kernel K eine d-dimensionale Matrix, im vorliegenden Beispiel eine zweidimensionale Matrix, die in der Regel klein ist im Vergleich zur Anzahl der Knoten 520, 522, beispielsweise eine 3x3 Matrix oder eine 5x5 Matrix. Dies bedeutet insbesondere, dass die Gewichte der Kanten in der Faltungsschicht 511 nicht unabhängig sind, sondern so gewählt werden, dass sie die besagte Faltungsgleichung ergeben. Insbesondere gibt es für einen Kernel, der eine 3x3-Matrix ist, nur 9 unabhängige Gewichte, wobei jeder Eintrag der Kernelmatrix einem unabhängigen Gewicht entspricht, unabhängig von der Anzahl der Knoten 520, 522 in der vorderen Knotenschicht 510 und der hinteren Knotenschicht 512.

Im Allgemeinen verwenden faltende neuronale Netzwerke 500 Knotenschichten 510, 512, 514 mit einer Vielzahl von Kanälen, insbesondere aufgrund der Verwendung einer Vielzahl von Kerneln in den Faltungsschichten 511. In diesen Fällen können die Knotenschichten als (d+1)-dimensionale Matrizen betrachtet werden, wobei die erste Dimension die Kanäle indiziert. Die Wirkung einer Faltungsschicht 511 ist dann in einem zweidimensionalen Beispiel definiert als ${x}_{b}^{\left(n\right)} \left[i, j\right] = {\sum }_{a} \left({K}_{a , b}∗{x}_{a}^{\left(n-1\right)}\left[i, j\right]={\sum }_{a} {\sum }_{i ′} {\sum }_{j ′} {K}_{a , b} \left[i′,j′\right] ⋅ {x}_{a}^{\left(n-1\right)} \left[i-i′,j-j′\right] ,\right.$ wobei ${x}_{a}^{\left(n\right)}$ dem a-ten Kanal der vorhergehenden Kotenschicht 510 entspricht, ${x}_{b}^{\left(n\right)}$ dem b-ten Kanal der nachfolgenden Knotenschicht 512 entspricht und *K_{a,b}* einem der Kernels entspricht. Wenn eine Faltungsschicht 511 auf eine vorhergehende Knotenschicht 510 mit A-Kanälen wirkt und eine nachfolgende Knotenschicht 512 mit B-Kanälen ausgibt, gibt es A·B unabhängige d-dimensionale Kernel *K_{a,b}.*

Im Allgemeinen können in faltenden neuronalen Netzen 500 Aktivierungsfunktionen verwendet werden. In dieser Ausführungsform wird ReLU (rectified linear unit) verwendet, mit R(z) = max(0, z), so dass die Wirkung der Faltungsschicht 511 in dem zweidimensionalen Beispiel $\begin{matrix}{x}_{b}^{\left(n\right)} \left[i, j\right] = R \left({\sum }_{a} \left({K}_{a , b}∗{x}_{a}^{\left(n-1\right)}\left[i, j\right]\right.\right) \\ = R \left({\sum }_{a} {\sum }_{i ′} {\sum }_{j ′} {K}_{a , b} \left[i′,j′\right] ⋅ {x}_{a}^{\left(n-1\right)} \left[i-i′,j-j′\right]\right)\end{matrix}$ ist. Es ist auch möglich, andere Aktivierungsfunktionen zu verwenden, beispielsweise ELU (Exponential Linear Unit), LeakyReLU, Sigmoid, Tanh oder Softmax.

In der angezeigten Ausführungsform beinhaltet die Eingabeschicht 510 36 Knoten 520, die in einer zweidimensionalen 6x6-Matrix angeordnet sind. Die erste verborgene Knotenschicht 512 beinhaltet 72 Knoten 522, die als zweidimensionale 6x6-Matrizen angeordnet sind, wobei jede der beiden Matrizen das Ergebnis einer Faltung der Werte der Eingabeschicht mit einem 3x3-Kern innerhalb der Faltungsschicht 511 ist. Äquivalent dazu können die Knoten 522 der ersten versteckten Knotenschicht 512 als eine dreidimensionale 2x6x6-Matrix interpretiert werden, wobei die erste Dimension der Kanaldimension entspricht.

Ein Vorteil der Verwendung von Faltungsschichten 511 besteht darin, dass eine räumlich lokale Korrelation der Eingabedaten ausgenutzt werden kann, indem ein lokales Konnektivitätsmuster zwischen den Knoten benachbarter Schichten erzwungen wird, insbesondere indem jeder Knoten nur mit einem kleinen Bereich der Knoten der vorhergehenden Schicht verbunden ist.

Eine Pooling-Schicht 513 ist eine Verbindungsschicht zwischen einer vorhergehenden Knotenschicht 512 mit Knotenwerten x(n-1) und einer nachfolgenden Knotenschicht 514 mit Knotenwerten x(n). Eine Pooling-Schicht 513 kann insbesondere durch die Struktur und die Gewichte der Kanten und die Aktivierungsfunktion charakterisiert sein, die eine Pooling-Operation auf der Grundlage einer nichtlinearen Pooling-Funktion f bilden. Beispielsweise können im zweidimensionalen Fall die Werte x(n) der Knoten 524 der nachfolgenden Knotenschicht 514 basierend auf den Werten x(n-1) der Knoten 522 der anterioren Knotenschicht 512 wie folgt berechnet werden ${x}_{b}^{\left(n\right)} \left[i, j\right] = f \left({x}_{b}^{\left(n-1\right)}\left[{id}_{1}, {jd}_{2}\right],…, {x}_{b}^{\left(n-1\right)}\left[\left(i+1\right){d}_{1}-1,\left(j+1\right){d}_{2}-1\right]\right) .$

Mit anderen Worten kann durch die Verwendung einer Pooling-Schicht 513 die Anzahl der Knoten 522, 524 reduziert werden, indem eine Anzahl d1-d2 benachbarter Knoten 522 in der vorhergehenden Knotenschicht 512 durch einen einzigen Knoten 522 in der nachfolgenden Knotenschicht 514 ersetzt wird, der als Funktion der Werte der genannten Anzahl benachbarter Knoten berechnet wird. Die Pooling-Funktion f kann insbesondere die Max-Funktion, der Mittelwert oder die L2-Norm sein. Insbesondere sind bei einer Pooling-Schicht 513 die Gewichte der eingehenden Kanten fest und werden durch das Trainieren nicht verändert.

Der Vorteil der Verwendung einer Pooling-Schicht 513 ist, dass die Anzahl der Knoten 522, 524 und die Anzahl der Parameter reduziert wird. Dies führt zu einer Reduzierung des Berechnungsaufwands im Netzwerk und zu einer Kontrolle der Überanpassung.

In der gezeigten Ausführungsform ist die Pooling-Schicht 513 eine Max-Pooling-Schicht, bei der vier benachbarte Knoten durch nur einen Knoten ersetzt werden, wobei der Wert das Maximum der Werte der vier benachbarten Knoten ist. Das Max-Pooling wird auf jede d-dimensionale Matrix der vorherigen Schicht angewendet. In dieser Ausführungsform wird das Max-Pooling auf jede der zweidimensionalen Matrizen angewandt, wodurch die Anzahl der Knoten von 72 auf 18 reduziert wird.

Im Allgemeinen können die letzten Schichten eines faltenden neuronalen Netzwerks 500 vollständig verbundene Schichten 515 sein. Eine vollständig verbundene Schicht 515 ist eine Verbindungsschicht zwischen einer vorhergehenden Knotenschicht 514 und einer nachfolgenden Knotenschicht 516. Eine vollständig verbundene Schicht 513 kann dadurch gekennzeichnet sein, dass eine Mehrheit, insbesondere alle Kanten zwischen den Knoten 514 der vorhergehenden Knotenschicht 514 und den Knoten 516 der nachfolgenden Knotenschicht vorhanden sind, und wobei das Gewicht jeder dieser Kanten individuell angepasst werden kann.

In dieser Ausführungsform sind die Knoten 524 der vorderen Knotenschicht 514 der vollständig verbundenen Schicht 515 sowohl als zweidimensionale Matrizen dargestellt, als auch zusätzlich als nicht zusammenhängende Knoten, die als eine Linie von Knoten angezeigt werden, wobei die Anzahl der Knoten zur besseren Darstellbarkeit reduziert wurde. Dieser Vorgang wird auch als Abflachung (englisch: flattening) bezeichnet. In dieser Ausführungsform ist die Anzahl der Knoten 526 in der nachfolgenden Knotenschicht 516 der vollständig verbundenen Schicht 515 kleiner als die Anzahl der Knoten 524 in der vorhergehenden Knotenschicht 514. Alternativ kann die Anzahl der Knoten 526 auch gleich oder größer sein.

Außerdem wird in dieser Ausführungsform die Softmax-Aktivierungsfunktion innerhalb der vollständig verbundenen Schicht 515 verwendet. Durch die Anwendung der Softmax-Funktion ist die Summe der Werte aller Knoten 526 der Ausgabeschicht 516 gleich 1, und alle Werte aller Knoten 526 der Ausgabeschicht 516 sind reelle Zahlen zwischen 0 und 1. Insbesondere bei Verwendung des faltenden neuronalen Netzwerks 500 zur Kategorisierung von Eingabedaten können die Werte der Ausgabeschicht 516 als die Wahrscheinlichkeit interpretiert werden, dass die Eingabedaten in eine der verschiedenen Kategorien fallen.

Insbesondere können faltende neuronale Netzwerke 500 basierend auf dem Backpropagation-Algorithmus trainiert werden. Um eine Überanpassung zu verhindern, können Verfahren der Regularisierung verwendet werden, beispielsweise das Auslassen von Knoten 520, ..., 524, stochastisches Pooling, die Verwendung künstlicher Daten, Gewichtsabfall basierend auf der L1- oder L2-Norm oder Max-Norm-Beschränkungen.

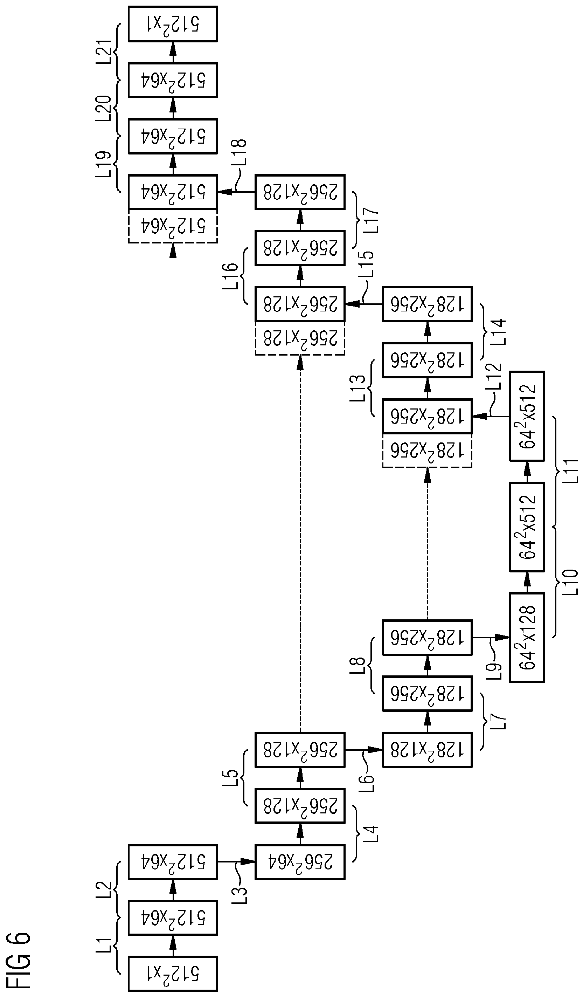

Im Beispiel von FIG 6 ist das MLM ein CNN mit einer U-Net-Struktur. In dem gezeigten Beispiel sind die Eingabedaten für das CNN ein zweidimensionales medizinisches Bild mit 512x512 Pixeln, wobei jedes Pixel einen Intensitätswert beinhaltet. Das CNN beinhaltet Faltungsschichten, die durch durchgezogene, horizontale Pfeile dargestellt sind, Pooling-Schichten, die durch durchgezogene, nach unten zeigende Pfeile dargestellt sind, und Upsampling-Schichten, die durch durchgezogene, nach oben zeigende Pfeile dargestellt sind. Die Anzahl der jeweiligen Knoten ist in den Kästen angegeben. Innerhalb der U-Net-Struktur werden zunächst die Eingabebilder heruntergetastet (englisch: downsampling), insbesondere durch Verkleinerung der Bilder und Erhöhung der Anzahl der Kanäle. Anschließend werden sie hochgerechnet(englisch: upsampling), insbesondere durch Vergrößerung der Bilder und Verringerung der Anzahl der Kanäle, um ein transformiertes Bild zu erzeugen.

Alle außer den letzten Faltungsschichten L1, L2, L4, L5, L.7, L8, L10, L11, L13, L14, L16, L17, L19, L20 verwenden 3x3-Kerne mit einem Padding von 1, die ReLU-Aktivierungsfunktion und eine Anzahl von Filtern oder Faltungskernen, die der Anzahl der Kanäle der jeweiligen Knotenschichten entspricht, wie in FIG 6 gezeigt. Die letzte Faltungsschicht verwendet einen 1x1-Kernel ohne Padding und die ReLU-Aktivierungsfunktion.

Die Pooling-Schichten L3, L6, L9 sind Max-Pooling-Schichten, die vier benachbarte Knoten durch nur einen Knoten ersetzen, wobei der Wert das Maximum der Werte der vier benachbarten Knoten ist. Die Upsampling-Schichten L12, L15, L18 sind transponierte Faltungsschichten mit 3x3-Kernen und Stride 2, wodurch sich die Anzahl der Knoten effektiv vervierfacht. Die gestrichelten horizontalen Pfeile entsprechen Verkettungsoperationen, bei denen die Ausgabe einer Faltungsschicht L2, L5, L8 des Downsampling-Zweigs der U-Net-Struktur als zusätzliche Eingaben für eine Faltungsschicht L13, L16, L19 des Upsampling-Zweigs der U-Net-Struktur verwendet wird. Diese zusätzlichen Eingabedaten werden als zusätzliche Kanäle in der Eingangsknotenschicht für die Faltungsschicht L13, L16, L19 des Upsampling-Zweigs behandelt.

Zum Trainieren des CNN wurde eine Datenbank mit 500 ersten medizinischen Bildern verwendet, wobei die jeweilige Segmentierungsmaske basierend auf Annotierungen von Radiologen-Experten erstellt wurde. Insbesondere bestimmten die Experten für jedes der 500 ersten medizinischen Bilder eine Segmentierungsmaske für eine Struktur von Interesse, wobei den Pixeln, die der Struktur von Interesse entsprechen, ein Wert von 1 zugeordnet wurde und den Pixeln, die der Struktur von Interesse nicht entsprechen, ein Wert von 0. Die Datenbank wurde in Trainingsdaten (320 Datensätze), Validierungsdaten (80 Datensätze) und Testdaten (100 Datensätze) aufgeteilt. Zum Trainieren des CNN wurde der Backpropagation-Algorithmus verwendet, basierend auf einer binären Kreuzentropie-Kostenfunktion $L \left(x, y\right) = {\sum }_{i} {\sum }_{j} BCE \left(y\left[i, j\right],M\left(x\right)\left[i, j\right]\right)$ mit $BCE \left(a, b\right) : = - a log \left(b\right) \left(b\right) - \left(1-a\right) log \left(1-b\right) ,$ wobei x ein erstes medizinisches Bild bezeichnet, y die entsprechende Segmentierungsmaske bestimmt, die von dem Radiologieexperten erstellt wurde, und M(x) das Ergebnis der Anwendung des CNN auf das erste medizinische Eingabebild x bezeichnet. Alternativ könnte man auch andere Kostenfunktionen wie gewichtete binäre Kreuzentropie, Focal Loss oder Dice Loss verwenden.

Basierend auf dem Validierungssatz von 80 Datensätzen und den entsprechenden Annotierungen wurde das Modell mit der besten Leistungsfähigkeit aus mehreren maschinellen Lernmodellen (mit unterschiedlichen Hyperparametern, beispielsweise Anzahl der Schichten, Größe und Anzahl der Kerne, Polsterung et cetera) ausgewählt. Die Spezifität und die Sensitivität wurden basierend auf dem Testsatz bestimmt, der 100 Datensätze und die entsprechenden Annotierungen beinhaltet.

Unabhängig vom grammatikalischen Geschlecht eines bestimmten Begriffes sind Personen mit männlicher, weiblicher oder anderer Geschlechteridentität mit umfasst.

## Patentansprüche

1. Röntgenbildgebungsverfahren, wobei
- wenigstens zwei Röntgenbilder (8a, 8b) eines abzubildenden Bereichs eines Objekts (5) erhalten werden, wobei die wenigstens zwei Röntgenbilder (8a, 8b) unterschiedlichen ersten Aufnahmezeiträumen entsprechen und eine Gefäßstruktur (13, 13a, 13b) des Objekts (5) in unterschiedlichen Kontrastmittelfüllzuständen darstellen;
- basierend auf den wenigstens zwei Röntgenbildern (8a, 8b) eine gemeinsame Gefäßmaske (10) erzeugt wird, welche die Gefäßstruktur (13, 13a, 13b) darstellt;
- für jedes der wenigstens zwei Röntgenbilder (8a, 8b) eine vorläufige Gefäßmaske erzeugt wird, indem ein trainiertes erstes Maschinenlernmodell auf erste Eingangsdaten, welche von dem jeweiligen Röntgenbild (8a, 8b) abhängen, angewendet wird und die gemeinsame Gefäßmaske (10) abhängig von den vorläufigen Gefäßmasken erzeugt wird,
- wenigstens zwei Livebilder des abzubildenden Bereichs, welche unterschiedlichen zweiten Aufnahmezeiträumen entsprechen, erhalten werden; und
- für jedes der wenigstens zwei Livebilder abhängig von der gemeinsamen Gefäßmaske (10) ein entsprechendes Überlagerungsbild (11) zur Anzeige auf einem Anzeigegerät erzeugt wird.

2. Röntgenbildgebungsverfahren nach Anspruch 1, wobei
- wenigstens ein Maskenbild (6) des abzubildenden Bereichs erhalten wird;
- wenigstens zwei Kontrastmittelbilder (7a, 7b) des abzubildenden Bereichs erhalten werden, wobei die wenigstens zwei Kontrastmittelbilder (7a, 7b) die Gefäßstruktur (13, 13a, 13b) in den unterschiedlichen Kontrastmittelfüllzuständen darstellen;
- für jedes der wenigstens zwei Kontrastmittelbilder (7a, 7b) basierend auf dem wenigstens einen Maskenbild (6) ein Subtraktionsbild (8a, 8b) erzeugt wird; und
- die wenigstens zwei Röntgenbilder (8a, 8b) durch die Subtraktionsbilder (8a, 8b) gegeben sind.

3. Röntgenbildgebungsverfahren nach Anspruch 2, wobei
- das wenigstens eine Maskenbild (6) wenigstens zwei Maskenbilder (6) enthält, wobei die wenigstens zwei Maskenbilder (6) unterschiedlichen weiteren Aufnahmezeiträumen entsprechen;
- für jedes der wenigstens zwei Kontrastmittelbilder (7a, 7b) ein entsprechendes Summenbild (9a, 9b) durch gewichtete Summation der wenigstens zwei Maskenbilder (6) erzeugt wird; und
- für jedes der wenigstens zwei Kontrastmittelbilder (7a, 7b) das jeweilige Subtraktionsbild (8a, 8b) durch Subtraktion des jeweiligen Summenbildes (9a, 9b) von dem jeweiligen Kontrastmittelbild (7a, 7b) erzeugt wird.

4. Röntgenbildgebungsverfahren nach einem der vorhergehenden Ansprüche, wobei
- die gemeinsame Gefäßmaske (10) gemäß einer vorgegebenen Regel als eine der vorläufigen Gefäßmasken ausgewählt wird; oder
- die gemeinsame Gefäßmaske (10) durch Anwendung eines trainierten zweiten Maschinenlernmodells auf zweite Eingangsdaten, welche von den vorläufigen Gefäßmasken abhängen, erzeugt wird.

5. Röntgenbildgebungsverfahren nach einem der vorhergehenden Ansprüche, wobei abhängig von der gemeinsamen Gefäßmaske (10) für jede der vorläufigen Gefäßmasken eine Registrierungsvorschrift ermittelt wird und für jedes der wenigstens zwei Livebilder
- durch Abgleich des jeweiligen Livebilds mit den wenigstens zwei Kontrastmittelbildern (7a, 7b) eines der Kontrastmittelbilder (7a, 7b) identifiziert wird; und
- zum Erzeugen des Überlagerungsbildes (11) die gemeinsame Gefäßmaske (10) anhand derjenigen Registrierungsvorschrift, welche dem identifizierten Kontrastmittelbild (7a, 7b) entspricht, zu dem jeweiligen Livebild registriert und dem jeweiligen Livebild überlagert wird.

6. Röntgenbildgebungsverfahren nach einem der Ansprüche 1 bis 4, wobei abhängig von der Gefäßmaske (10) für jede der vorläufigen Gefäßmasken eine Registrierungsvorschrift ermittelt wird und für jedes der wenigstens zwei Livebilder
- durch Abgleich des jeweiligen Livebilds mit den wenigstens zwei Röntgenbildern (8a, 8b) eines der wenigstens zwei Röntgenbilder (8a, 8b) identifiziert wird; und
- zum Erzeugen des Überlagerungsbildes (11) die gemeinsame Gefäßmaske (10) anhand derjenigen Registrierungsvorschrift, welche dem identifizierten Röntgenbild (8a, 8b) entspricht zu dem jeweiligen Livebild registriert und dem jeweiligen Livebild überlagert wird.

7. Röntgenbildgebungsverfahren nach einem der vorhergehenden Ansprüche, wobei das erste Maschinenlernmodell ein faltendes neuronales Netzwerk oder ein Transformernetzwerk beinhaltet.

8. Röntgenbildgebungsverfahren nach einem der vorhergehenden Ansprüche, wobei die wenigstens zwei Röntgenbilder (8a, 8b) während einer Kontrastmittelverabreichung in die Gefäßstruktur (13, 13a, 13b) erzeugt werden.

9. Röntgenbildgebungsverfahren nach einem der vorhergehenden Ansprüche, wobei die wenigstens zwei Röntgenbilder (8a, 8b) die Gefäßstruktur (13, 13a, 13b) während unterschiedlicher Bewegungszustände des Objekts (5) darstellen.

10. Röntgenbildgebungsverfahren nach einem der vorhergehenden Ansprüche, wobei wenigstens eines der wenigstens zwei Livebilder ein Werkzeug zur Gefäßintervention in dem abzubildenden Bereich darstellt.

11. Datenverarbeitungsvorrichtung (2) aufweisend wenigstens eine Recheneinheit, die angepasst ist, ein Röntgenbildgebungsverfahren nach einem der vorhergehenden Ansprüche durchzuführen.

12. Röntgenbildgebungssystem (1) aufweisend eine Röntgenquelle (4) und einen Röntgendetektor (3) zum Erzeugen der wenigstens zwei Livebilder sowie eine Datenverarbeitungsvorrichtung (2) nach Anspruch 11.

13. Computerprogrammprodukt mit Befehlen, die bei Ausführung durch eine Datenverarbeitungsvorrichtung (2) die Datenverarbeitungsvorrichtung (2) dazu veranlassen, ein Röntgenbildgebungsverfahren nach einem der Ansprüche 1 bis 10 durchzuführen.

## Claims

1. X-ray imaging method, wherein
- at least two X-ray images (8a, 8b) are obtained of a region to be depicted in an object (5), wherein the at least two X-ray images (8a, 8b) correspond to different first acquisition time-frames and represent a vascular structure (13, 13a, 13b) of the object (5) in different contrast-agent filling states;
- a shared vessel mask (10), which represents the vascular structure (13, 13a, 13b), is generated on the basis of the at least two X-ray images (8a, 8b);
- for each of the at least two X-ray images (8a, 8b) a provisional vessel mask is generated by applying a trained first machine-learning model to first input data, which depends on the particular X-ray image (8a, 8b); and the shared vessel mask (10) is generated depending on the provisional vessel masks,
- at least two live images of the region to be depicted are obtained, which correspond to different second acquisition time-frames; and
- for each of the at least two live images, depending on the shared vessel mask (10), a corresponding overlay image (11) for displaying on a display device is generated.

2. X-ray imaging method according to claim 1, wherein
- at least one mask image (6) of the region to be depicted is obtained;
- at least two contrast-agent images (7a, 7b) of the region to be depicted are obtained, wherein the at least two contrast-agent images (7a, 7b) represent the vascular structure (13, 13a, 13b) in the different contrast-agent filling states;
- for each of the at least two contrast-agent images (7a, 7b) a subtraction image (8a, 8b) is generated on the basis of the at least one mask image (6); and
- the at least two X-ray images (8a, 8b) are given by the subtraction images (8a, 8b).

3. X-ray imaging method according to claim 2, wherein
- the at least one mask image (6) contains at least two mask images (6), wherein the at least two mask images (6) correspond to different further acquisition time-frames;
- for each of the at least two contrast-agent images (7a, 7b) a corresponding summation image (9a, 9b) is generated by weighted summation of the at least two mask images (6); and
- for each of the at least two contrast-agent images (7a, 7b) the associated subtraction image (8a, 8b) is generated by subtracting the associated summation image (9a, 9b) from the particular contrast-agent image (7a, 7b).

4. X-ray imaging method according to one of the preceding claims, wherein
- the shared vessel mask (10) is selected as one of the provisional vessel masks according to a defined rule; or
- the shared vessel mask (10) is generated by applying a trained second machine-learning model to second input data, which depends on the provisional vessel masks.

5. X-ray imaging method according to one of the preceding claims, wherein a registration instruction is determined for each of the provisional vessel masks depending on the shared vessel mask (10), and for each of the at least two live images
- one of the contrast-agent images (7a, 7b) is identified by comparing the particular live image with the at least two contrast-agent images (7a, 7b); and
- for the purpose of generating the overlay image (11), the shared vessel mask (10) is registered with the particular live image by means of the registration instruction that corresponds to the identified contrast-agent image (7a, 7b), and overlaid on the particular live image.

6. X-ray imaging method according to one of claims 1 to 4, wherein a registration instruction is determined for each of the provisional vessel masks depending on the vessel mask (10), and for each of the at least two live images
- one of the at least two X-ray images (8a, 8b) is identified by comparing the particular live image with the at least two X-ray images (8a, 8b); and
- for the purpose of generating the overlay image (11), the shared vessel mask (10) is registered with the particular live image by means of the registration instruction that corresponds to the identified X-ray image (8a, 8b), and overlaid on the particular live image.

7. X-ray imaging method according to one of the preceding claims, wherein the first machine-learning model includes a convolutional neural network or a transformer network.

8. X-ray imaging method according to one of the preceding claims, wherein the at least two X-ray images (8a, 8b) are generated during administration of a contrast agent into the vascular structure (13, 13a, 13b).

9. X-ray imaging method according to one of the preceding claims, wherein the at least two X-ray images (8a, 8b) represent the vascular structure (13, 13a, 13b) during different movement states of the object (5).

10. X-ray imaging method according to one of the preceding claims, wherein at least one of the at least two live images represents an instrument for vascular intervention in the region to be depicted.

11. Data processing apparatus (2) having at least one computing unit, which is adapted to perform an X-ray imaging method according to one of the preceding claims.

12. X-ray imaging system (1) having an X-ray source (4) and an X-ray detector (3) for generating the at least two live images, and having a data processing apparatus (2) according to claim 11.

13. Computer program product comprising commands which, on execution by a data processing apparatus (2), cause the data processing apparatus (2) to perform an X-ray imaging method according to one of claims 1 to 10.

## Revendications

1. Procédé d'imagerie par rayons X, dans lequel
- on obtient au moins deux images (8a, 8b) par rayons X d'une partie à représenter d'un objet (5), dans lequel les au moins deux images (8a, 8b) par rayons X correspondent à des premiers laps de temps d'enregistrement différents et représentent une structure (13, 13a, 13b) de vaisseau de l'objet (5) dans des états différents de remplissage par un agent de contraste ;
- sur la base des au moins deux images (8a, 8b) par rayons X, on produit un masque (10) de vaisseau commun, qui représente la structure (13, 13a, 13b) du vaisseau ;
- pour chacune des au moins deux images (8a, 8b) par rayons X, on produit un masque de vaisseau provisoire en appliquant un premier modèle d'apprentissage automatique entraîné à de premières données d'entrée, qui dépendent de l'image (8a, 8b) par rayons X respective, et en produisant le masque (10) de vaisseau commun en fonction des masques de vaisseau provisoires,
- on obtient au moins deux images en direct de la partie à représenter, qui correspondent à des deuxièmes laps de temps de réception différents ; et
- pour chacune des au moins deux images en direct, on produit en fonction du masque (10) de vaisseau commun, une image (11) de superposition correspondante pour affichage sur un appareil d'affichage.

2. Procédé d'imagerie par rayons X suivant la revendication 1, dans lequel
- on obtient au moins une image (6) de masque de la partie à représenter ;
- on obtient au moins deux images (7a, 7b) par agent de contraste de la partie à représenter, dans lequel les au moins deux images (7a, 7b) par agent de contraste représentent la structure (13, 13a, 13b) du vaisseau dans les états différents de remplissage par un agent de contraste ;
- pour chacune des au moins deux images (7a, 7b) par agent de contraste, on produit une image (8a, 8b) de soustraction sur la base de la au moins une image (6) de masque ; et
- les au moins deux images (8a, 8b) par rayons X sont données par les images (8a, 8b) de soustraction.

3. Procédé d'imagerie par rayons X suivant la revendication 2, dans lequel
- la au moins une image (6) de masque contient au moins deux images (6) de masque, dans lequel les au moins deux images (6) de masque correspondent à d'autres laps de temps d'enregistrement différents ;
- pour chacune des au moins deux images (7a, 7b) par agent de contraste, on produit une image (9a, 9b) somme correspondante par sommation pondérée des au moins deux images (6) de masque ; et
- pour chacune des au moins deux images (7a, 7b) par agent de contraste, on produit l'image (8a, 8b) respective de soustraction en soustrayant l'image (9a, 9b) somme respective de l'image (7a, 7b) par agent de contraste respective.

4. Procédé d'imagerie par rayons X suivant l'une des revendications précédentes, dans lequel
- on sélectionne le masque (10) de vaisseau commun suivant une règle donnée à l'avance comme l'un des masques de vaisseau provisoires ;
ou
- on produit le masque (10) de vaisseau commun en appliquant un deuxième modèle d'apprentissage automatique entraîné à des deuxièmes données d'entrée, qui dépendent des masques de vaisseau provisoires.

5. Procédé d'imagerie par rayons X suivant l'une des revendications précédentes, dans lequel, en fonction du masque (10) de vaisseau commun, on établit pour chacun des masques de vaisseau provisoires une prescription de correspondance et pour chacune des au moins deux images directes,
- par égalisation de l'image directe respective au au moins deux images (7a, 7b) par agent de contraste, on identifie l'une des images (7a, 7b) par agent de contraste ; et
- pour la production de l'image (11) de superposition, on fait correspondre le masque (10) de vaisseau commun à l'aide de la prescription de correspondance, qui correspond à l'image (7a, 7b) par agent de contraste identifiée à l'image directe respective et on le superpose à l'image directe respective.

6. Procédé d'imagerie par rayons X suivant l'une des revendications 1 à 4, dans lequel, en fonction du masque (10) de vaisseau, on établit pour chacun des masques de vaisseau provisoires une prescription de correspondance et pour chacune des au moins deux images directes
- par égalisation de l'image directe respective avec les au moins deux images (8a, 8b) par rayons X, on identifie l'une des au moins deux images (8a, 8b) par rayons X ; et
- pour la production de l'image (11) de superposition, on fait correspondre le masque (10) de vaisseau commun à l'aide de la prescription de correspondance respective, qui correspond à l'image (8a, 8b) par rayons X identifiée à l'image directe respective et on le superpose à l'image directe respective.

7. Procédé d'imagerie par rayons X suivant l'une des revendications précédentes, dans lequel le premier modèle d'apprentissage automatique comporte un réseau neuronal convolutif ou un réseau transformeur.

8. Procédé d'imagerie par rayons X suivant l'une des revendications précédentes, dans lequel on produit les au moins deux images (8a, 8b) par rayons X pendant une administration d'agent de contraste dans la structure (13, 13a, 13b) de vaisseau.

9. Procédé d'imagerie par rayons X suivant l'une des revendications précédentes, dans lequel les au moins deux images (8a, 8b) par rayons X représentent la structure (13, 13a, 13b) de vaisseau pendant des états de mouvement différents de l'objet (5).

10. Procédé d'imagerie par rayons X suivant l'une des revendications précédentes, dans lequel au moins l'une des au moins deux images directes représentent un outil d'intervention sur un vaisseau dans la partie à représenter.

11. Dispositif (2) de traitement de données comportant au moins une unité informatique, qui est adaptée pour effectuer un procédé d'imagerie par rayons X suivant l'une des revendications précédentes.

12. Système (1) d'imagerie par rayons X comportant une source (4) de rayons X et un détecteur (3) de rayons X pour la production d'au moins deux images directes ainsi qu'un dispositif (2) de traitement de données suivant la revendication 11.

13. Produit de programme d'ordinateur comprenant des instructions, qui, lors de l'exécution par un dispositif (2) de traitement de données, font que le dispositif (2) de traitement de données exécute un procédé d'imagerie par rayons X suivant l'une des revendications 1 à 10.
